(19) Europäisches Patentamt / European Patent Office / Office européen des brevets

(11) **EP 3 304 377 B1**

(12) **EUROPEAN PATENT SPECIFICATION**

(45) Date of publication and mention of the grant of the patent:
**15.01.2020 Bulletin 2020/03**

(21) Application number: **16731046.5**

(22) Date of filing: **08.06.2016**

(51) Int Cl.:
*G16B 20/00* [(2019.01)]

(86) International application number:
**PCT/EP2016/063067**

(87) International publication number:
**WO 2016/198471 (15.12.2016 Gazette 2016/50)**

(54) **METHOD FOR DETERMINING DROUGHT TOLERANCE IN MAIZE**

VERFAHREN ZUR BESTIMMUNG DER DÜRRETOLERANZ BEI MAIS

PROCÉDÉ PERMETTANT DE DÉTERMINER LA TOLÉRANCE À LA SÉCHERESSE DANS LE MAÏS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.06.2015 EP 15305867**
**23.07.2015 PCT/IB2015/001372**

(43) Date of publication of application:
**11.04.2018 Bulletin 2018/15**

(73) Proprietor: **Limagrain Europe**
**63360 Saint-Beauzire (FR)**

(72) Inventors:
• **MURIGNEUX, Alain**
**63670 La Roche Blanche (FR)**
• **HENRIOT, Fabienne**
**63400 Chamaliere (FR)**
• **PERSONNE, Manuel**
**63720 Chappes (FR)**
• **RENAULT, Morgan**
**49630 Maze (FR)**
• **DELLUC, Caroline**
**63720 Chappes (FR)**
• **DEBEUF, Roland**
**63720 Chappes (FR)**
• **BOYARD, Chloé**
**63540 Saulzet-Le-Chaud (FR)**

(74) Representative: **Regimbeau**
**87 rue de Sèze**
**69477 Lyon Cedex 06 (FR)**

(56) References cited:
• CHAPUIS R ET AL: "Resiliences to water deficit in a phenotyping platform and in the field: How related are they in maize?", EUROPEAN JOURNAL OF AGRONOMY, ELSEVIER, AMSTERDAM, NL, vol. 42, 21 December 2011 (2011-12-21), pages 59-67, XP028400543, ISSN: 1161-0301, DOI: 10.1016/J.EJA.2011.12.006 [retrieved on 2012-01-16]
• F. TARDIEU ET AL: "Genetic and Physiological Controls of Growth under Water Deficit", PLANT PHYSIOLOGY., vol. 164, no. 4, 1 April 2014 (2014-04-01) , pages 1628-1635, XP055226058, US ISSN: 0032-0889, DOI: 10.1104/pp.113.233353
• C. WELCKER ET AL: "A Common Genetic Determinism for Sensitivities to Soil Water Deficit and Evaporative Demand: Meta-Analysis of Quantitative Trait Loci and Introgression Lines of Maize", PLANT PHYSIOLOGY., vol. 157, no. 2, 1 October 2011 (2011-10-01), pages 718-729, XP055226128, US ISSN: 0032-0889, DOI: 10.1104/pp.111.176479
• E. M. PARDO ET AL: "Drought Tolerance Screening Under Controlled Conditions Predicts Ranking of Water-Limited Yield of Field-Grown Soybean Genotypes", JOURNAL OF AGRONOMY AND CROP SCIENCE, vol. 201, no. 2, 3 April 2015 (2015-04-03) , pages 95-104, XP055226055, ISSN: 0931-2250, DOI: 10.1111/jac.12106
• ROBERTO TUBEROSA: "Phenotyping for drought tolerance of crops in the genomics era", FRONTIERS IN PHYSIOLOGY, vol. 3, 1 January 2012 (2012-01-01), XP055226050, CH ISSN: 1664-042X, DOI: 10.3389/fphys.2012.00347

**Description**

## INTRODUCTION

Scarcity of water is a severe environmental constraint to plant productivity. Drought-induced loss in crop yield probably exceeds losses from all other causes, since both the severity and the duration of the stress are critical. Indeed, drought is one of the major limitations to maize production worldwide - 15% of the world's maize crop production is lost every year due to drought. Plant response to water stress is dependent on the amount of water deficit, the intensity of the deficit, the duration of the drought stress, the plant variety/species under consideration, the developmental stage of the plant when the stress occurs, and other environmental variables such as temperature, relative humidity etc.

It is imperative to improve the drought tolerance of crops under the current changing weather circumstances. Currently, there is no economically viable technological means to facilitate crop production under drought. The development of crop plants tolerant to drought stress might be a promising approach, helping in meeting the food demands. Drought tolerance, however, is one of the most complex trait to breed for. The pattern (combinations of heat and drought), the timing in relation to the plant phenological stages and the intensity of the stress can all vary considerably. More specifically, drought stress is highly variable in its timing, duration and severity, and this result in high genotype by environment interaction variation. In addition, the whole-plant response to stress is complex. It is determined by component traits that interact and differ in their individual responses to the intensity and duration of water deficits and high temperature.

Various approaches have been used to assess drought stress tolerance in plants (see e.g., Chapuis *et al.*, 2012). The most common has been to perform cycles of selection among elite genotypes, evaluated for performance in both wet and dry environments. Breeding relies on phenotyping on large trials locations networks, representing every year a large set of environmental conditions. Long term selection on these diverse sets of environments contributes to the selection of stable genotypes, characterized by a better drought tolerance. This has been extremely efficient, as shown by the steady progress in grain yield in both well-watered and deficit conditions (Bolanos and Edmeades, 1993; Bruce *et al.*, 2002; Duvick, 2005). Other approaches involving *in silico* selection and plant modelling have also been used. They are time saving and offer opportunities for evaluating the performance of genotypes in multiple drought scenarios (Messina *et al.*, 2011). Nevertheless, these approaches require the modelling of the phenotype traits implied in stress tolerance accounting for environmental conditions and specific genotypes characteristics (Messina *et al.*, 2011; Tardieu and Tuberosa, 2010, Tardieu *et al.*, 2014, Welcker *et al.*, 2011).

A previous study (Chapuis *et al.*, 2012) aimed at assessing corn hybrids for drought tolerance using the correlation between the level of stress and the seed number per plant. Only the stress index calculated during the flowering period was taken into account. The correlation between the level of stress and the yield was found as non-significant. Thus there is still a need for a method for determining drought stress tolerance which can be used in breeding. The drought stress includes all related abiotic stress such as soil water deficit, air water deficit, heat stress, light radiation etc.

## DESCRIPTION

The present inventors have now devised a new method for determining drought stress tolerance in plant, especially in maize. This method is based on a direct estimation of resilience in the field during specific phases of the plant cycle. The inventors have found that a measure of plant behavior such as yield reflects the intensity of the stress undergone by the plant during specific phenological phases. A statistical relationship is drawn between grain yield and the stress index. In fact, the inventors have found that those two variables are linked through a linear regression, of which the slope represents the drought tolerance.

Thus in a first aspect, the present invention provides a method for determining drought stress tolerance of a plant line, wherein said drought stress tolerance is obtained by computing a linear regression between the grain yield of said plant line and a stress index. According to the invention, drought stress tolerance is given by the slope of the regression: the more tolerant a plant line, the closer to 0 the slope. In contrast, a highly negative slope indicates that the plant line is sensitive to drought stress.

Multiple field trials are needed to achieve the method of the invention. Preferably, said plant line is grown at multiple locations. By "multiple locations", it is herein referred to at least 2, preferably at least 3, more preferably at least 4, more preferably at least 5, more preferably at least 6, more preferably at least 7, most preferably at least 8 locations. Still more preferably, said plant lines is sown in at least 8 locations with 2 locations with IT > 80 and 2 locations with IT < 80 (IT is the stress index for soil water deficit and is further defined hereafter). But the line can also be sowed in a unique location but on successive years.

In a further preferred embodiment, said multiple locations are geographically separated. By "geographically separated", it is herein meant that at least 2 of these locations are separated by 100 Km, 200 km, 300 km, 400 km or more. Precocity area are defined for maize, the locations should be on the same precocity area or an precocity area compatible with the studied line.

When said plant line is grown at multiple locations, the variety of stresses encountered by said plant line, as well as the intensity thereof, is increased. According to this embodiment, the grain yield is measured for each location; likewise, the stress index is determined for each location: the regression between the yield and the stress index is drawn over all the locations where the plant line was grown. Every single location is thus taken advantage of to evaluate said plant line for stress tolerance. This leads to a regression relationship between grain yield and stress index which is based on a wide range of stresses, thus increasing the significance of the drought tolerance determination. The grain yield can be expressed in q/ha (q means quintal which correspond to 100kg and ha means hectare) but also as a percentage of the grain yields of checks lines grown on the same locations. Using the grain yield as a percentage of the checks leads to reduce the noises due to biotic or abiotic stresses other than drought.

In a preferred embodiment, the invention relates to a method for determining the drought tolerance of a plant line, said method comprising the steps of:

a) sowing said plant line in multiple locations;
b) determining a drought stress index for each location;
c) measuring grain yield of said plant line for each location;
d) computing a linear regression between said stress index of step b) and said grain yield of step c);
e) determining drought tolerance based on said regression.

The expression "drought tolerance " is synonym of "drought stress tolerance" in the context of the invention, and may also be named as "drought tolerance score" or "stress tolerance score", "drought tolerance value" or "stress tolerance value", "drought tolerance slope" or "stress tolerance slope". More preferably this value/slope/score is negative.

The term "drought stress", as used herein, refers to a condition without normal watering in plant growth, which is utilized as a very common term including all kind of abiotic stresses that induce harmful effects on plant growth and survival, for example "drought stress" as used herein includes such stresses as e.g., soil water deficit, vapor pressure deficit, heat stress or light radiation. More specifically, the term "drought" and "water-deficit" refers to environmental conditions where the amount of water (e.g., rainfall or other available water source for plant life) is less than the average water conditions for the particular environment, or the amount of water available is less than the amount of water typically needed by a certain species of plant or by a plant growing in a particular environment. Typically a "drought" indicates a more intense or more prolonged period of reduced water availability than a "water-deficit". The terms "drought-resistance" or "drought-tolerance" refer to the ability of a plant to recover from periods of drought stress (i.e., little or no water for a period of days). In the context of the presence invention, drought tolerance refers to the ability of a plant to achieve a yield performance as close as possible to the optimal yield whatever the intensity and the duration of the stress.

The effects of drought range from the morphological to the molecular levels. They are mostly evident at critical phenological stages such as flowering and grain filling. Drought stress in particular impacts grain yield. As used herein, "grain yield" refers to the grain weight for a cultivated surface at 15% moisture. The "seed number" as used herein refers to the number of seeds per plant. The "thousand kernel weight" (also referred to as the 1,000 kernel weight, 1,000 K weight or TKW) is the weight in grams of 1,000 seeds and is a well-known parameter in agriculture. TKW is measured with grain moisture content comprised between 10 and 20 %, preferably 15 %.

Seed number would be a good indicator of the yield if the TKW was not variable between genotypes. However, as demonstrated by the inventors, different genotypes can display highly disparate TKW. This means that, just like the grain number per plant, TKW is an important component to explain the grain yield. Chapuis *et al.* (2012) based their assessment of drought tolerance on a measure of the seed number only. In contrast, the method of the present invention yields a result which is more significant and more reliable.

As used herein, the "stress index" refers to a quantification of the drought stress undergone by the plant for a specific location. The stress index is thus an indication of the intensity of the drought stress undergone by the plant at said location. This can be evaluated by measuring at least one environmental parameter, said parameter being indicative of the stress applying to the plant at a given time at this location. The stress index is the average of said measurement of said at least one environmental parameter for at least one period of time. Examples of environmental parameters which may be used for measuring the stress index are but not limited to: soil water index, vapor pressure deficit, heat stress and light radiation.

According to the invention, the computing of a linear regression between the grain yield and a stress index for determining the drought stress tolerance of a plant line is carried out using a linear regression with fixed effects only or with random effects.

Fixed effects according to the present invention represent the stress index and the grain yield accurately determined or measured for each location of the sown maize line in step a).

According to a preferred embodiment, the linear regression in step d) is a linear regression with random effect(s), which preferably integrate information from the relative(s) of said maize line.

In this embodiment, not fixed effects only are used but rather a random effect to draw said regression and to calculate the slope of said regression. Preferably, the random effect model of the linear regression allows providing BLUP (Best Linear Unbiased Prediction) estimates for the slope and integrating information from the relative(s) of said maize line.

Using a random effect model in step (d) including information from these relatives, allows to correct the grain yield measured in step (c) into "an adjusted yield", thus leading to also correct the drought tolerance determined in step e) into "an adjusted drought tolerance" including molecular markers or pedigree information.

Random regression models are statistical models well known by the one skilled in the art, and for example as described by Schaeffer, 2004 and Lillehammer *et al.*, 2007.

As used herein, the term "relatives" means any plant or line with some degree of genetic relatedness. Relatives can be determined by their pedigree and/or by molecular markers.

As used herein, the expression "information from the relatives" means all the phenotypic record(s) obtained in previous experiments about these relatives (such as grain yield and stress index), as well as the pedigree and/or molecular markers.

Therefore, according to an advantageous embodiment, the linear regression in step d) of the method for determining the drought tolerance is a linear regression with random effect(s) which integrate information from the relative(s) of said maize line including phenotypic records as well as molecular markers and/or pedigree.

The phenological stage of the plant is known to be important for assessing the effects of water deprivation and other abiotic stress on said plant. For example, maize is particularly sensitive to stress from the 12-leave stage, ca. 3-4 weeks before flowering. In particular, the period around flowering time is known to be the most sensitive period to drought for maize. In a first embodiment, the at least one environmental parameter is measured at regular intervals during a period around the flowering time. Regular intervals can be of 10, 20, 30 min or 1, 2, or more hours, to 1 to 3 days.

According to a preferred embodiment, the flowering stress index is given by the formula:

$$\text{EP\_Flowering} = \frac{1}{n} \sum_{t=Init}^{t=End} EP_t$$

wherein "Init" is the beginning and "End" the end of the flowering period, "EPt" the value of the environmental parameter at the time unit t, and "n" the number of time units of the period.

The flowering time can be recorded easily by any method known to the person of skills in the art. Preferably, the "flowering time", as used herein, refers to the date at which 50% of the plants show silking.

The period around the flowering time wherein said at least one environmental parameter can be defined in any unit commonly used by the person of skills in the art. Preferably, said period is defined in degree days. As used herein, the "degree day" refers to the accumulated product of time and temperature between the developmental thresholds for each day. Said developmental threshold are well known to the person of skills in the art and part of the common general knowledge of said person. Methods of calculation of degree days are well-known to the person of skills in the art and will not be detailed here.

In a preferred embodiment, said period begins $n_1$ degree days before said flowering time and ends $n_2$ degree days after flowering time. According to this embodiment, the at least one environmental parameter is measured during a period beginning $n_1$ degree days before flowering time and ending $n_2$ degree days after flowering time, wherein $n_1$ and $n_2$ are two integers comprised between 70 and 120. Preferably, $n_1$ = 80 degree days and $n_2$ = 110 degree days. In this case, "Init", in equation 1, corresponds to the flowering date minus 80 degree days and "End" to the flowering date plus 110 degree days.

In some situations depending on the locations or year effects, the drought stress occurs later and the most critical period is at grain filling. Whereas the prior art only relied on the flowering time for determining the stress index (see e.g. Chapuis et al., 2012), the present inventors have shown that the grain filling time period is also important when determining the stress index.

Thus, in another embodiment, the at least one environmental parameter is measured at regular intervals during a period around the grain filling time.

According to a preferred embodiment, the grain filling stress index is then given by the formula:

$$\text{EP\_Filling} = \frac{1}{n} \sum_{t=Init}^{t=End} EP_t$$

wherein "Init" is the beginning and "End" the end of the grain filling period, "EPt" the value of the environmental parameter at the time unit t, and "n" the number of time units of the period.

The grain filling period begins with successful pollination and initiation of kernel development. Preferably, the grain filling period begins when the flowering period as defined above ends. In other words, according to this embodiment, the grain filling period starts at $n_2$ degree days after flowering time. The grain filling period has been sometimes described as ending with the apparition of the back spot. As used herein, the end of the grain filling period is preferably defined as the grain abortion limit stage. This stage corresponds to the stage when the final number of grains is defined, i.e., when endosperm cell multiplication is complete. Said stage is reached 550 degree days after flowering, but in the context of the invention the grain filling period should also end with the apparition of the black spot.

It is difficult to know whether drought will occur during the flowering period or during the grain filling period. Because of this uncertainty, the stress index based on either one of these two periods may not reflect accurately the situation. On the other hand, a stress index based on weighted combination of both periods is more reliable, as shown by the inventors.

According to a preferred embodiment, the at least one environmental parameter is measured at regular intervals during a period around the flowering time and during a period around the grain filling time.

According to a preferred embodiment, the stress index is then given by the formula:

$$\text{Stress Index} = W\_EP = \alpha * EP\_Flowering + \beta * EP\_Filling$$

wherein EP_Flowering and EP_Filling are as defined above, and $\alpha$ and $\beta$ are the weights of the combination, with $\alpha + \beta = 1$. Preferably, $\alpha$ and $\beta$ are chosen so as to take into account the differences of importance of drought in respect of each period. Depending on the specific environmental conditions, flowering may or may not be the most critical phenological stage for sensitivity to drought stress. In particular, since flowering is the most critical phenological stage for sensitivity to drought, it is preferred to choose $\alpha$ and $\beta$ so that $\alpha > \beta$, with $\alpha + \beta = 1$. Most preferably, $\alpha = 2/3$ and $\beta = 1/3$ if drought occurs principally at flowering. In another preferred embodiment, $\alpha = 2/3$ and $\beta = 1/3$ if stress occurs principally at grain filling.

The value of $\alpha$ and $\beta$ can also be adjusted for every year of experiment. A calculation of the drought stress index for a year of experiment can be done on a sample of known lines while determining the best $\alpha$ and $\beta$ value for this year of experiment.

This determination may rely on statistic methodologies known to the skilled person to define the $\alpha$ and $\beta$ values that optimize the regression between the yield and the stress index. The criterion for optimization is the maximization of the $R^2$ of the regressions of the yield over the stress index for the sample of known lines.

This drought stress index can also be calculated for a sample of lines and a crop model can be used to help in determining the best $\alpha$ and $\beta$ values to apply on the full experiment.

The environmental parameter used in the method of the invention is any parameter known to affect water supply to plants. In a preferred embodiment, said environmental parameter is selected in list consisting of the soil water index, the vapor pressure deficit, the heat stress, and the light radiation.

As used herein, the "soil water index" refers to the amount of water associated with a given volume or mass of soil. The computation of this amount of water will take into account the chemical and physical characteristics of the soil such as the structure, the texture or the organic matter content as well as the depth of the soil. In a soil saturated with water, water molecules are present in the pore spaces, as well as coating the soil particles. Water is thus easily available for plant roots to get water and the soil moisture tension is low. As water in the pores decreases, the suction or tension that the roots need to apply to get moisture increases. When soil tension reaches a certain threshold, the plant can no longer extract water from the soil even if there is water present. At this point the plant become stressed, begins to wilt and may eventually die if water is not replenished. A variety of methods and devices can be used to measure soil-water. These include the feel method, gravitational method, tensiometer, electrical resistance blocks, neutron probe, Phene cells, and time domain reflectometer. All these methods and devices are well known to the person of skills in the art and need not be detailed here (e.g., one can use Watermark Soil Moisture sensors, ref MONPACAN (supplied by Challenge Agriculture) associated to a Monitor, ref Monitor or Monitor Watermark GPRS R2-DX and Spiral gauge 25/22mm).

It is also possible to estimate the soil water status by the simulation of the soil water balance. This may be done using generic equations modelling on one end, the evapotranspiration of the crops and on the other end, the water available for the crops in the plant based on the soil characteristics.

As used herein, "vapor pressure deficit" (VPD) refers to the difference (deficit) between the amount of moisture in the air ($e_a$) and how much moisture the air can hold when it is saturated. The maximum water holding capacity (also called the dew point, $e_{sat}$ (Ta) increases with temperature. Adding moisture to air beyond it leads to deposition of water (dew). Higher VPD increases the transpirational demand, influencing how much moisture from plant tissues is transferred into the air. Low VPD indicates closer proximity to the dew point, meaning wet air. VPD can be measured with VPD sensors, which are in essence the combination of a Relative Humidity sensor and a temperature sensor in the same device. Such devices are commercially available (e.g., EL-USB-2 commercialized by Lascar Electronics, Whiteparish, UK; Davis Thermometers Radiation Shield, ref 7714, supplied by SARL DREANO, www.meteo-shopping.fr) and need not be further detailed here.

As used herein, "heat stress" refers to a modified temperature level sufficient to substantially and irreversibly affect plant growth and development. A "modified" temperature level can be the result of an increase or a decrease of said temperature. Indeed, "heat stress" in the domain of the invention can also refers to any stress related to temperature and should thus be understood as encompassing cold stress. Heat stress causes multifarious, and often adverse, alterations in plant growth, development, physiological processes, and yield. For example, high day temperatures can injure plants directly by causing hot tissue temperatures, or indirectly by creating plant-water deficits that arise due to high transpiration demands. Temperature can be easily monitored using any means known to the skilled person.

As used herein, light radiation refers to the intensity of the solar radiation. Especially during very cloudy days the level of radiation may become sub-optimal. The cumulated radiation over day is also influenced by day length (seasonal or latitude effect). For example light radiation may be measured in watt (W) or joules (J) per $m^2$ with a pyranometer or a light meter.

As detailed above, a grain yield and a stress index corresponding to an environmental parameter are obtained for the plant line tested for each of the locations. Based on the results from all locations, a regression is drawn between grain yield and stress index.

Preferably, the regression is given by the equation:

$$\text{Grain Yield} = \mu + a * W\_EP$$

wherein $\mu$ is the yield for a stress index equal to 0, equivalent to the potential yield of the given hybrid in absence of stress (as example, this value may fluctuate between 101 and 150 q/ha for commercial hybrids FAO 400-500 grown in France), and "a" is the drought tolerance.

Any appropriate software (e.g., the free software R, www.r-project.org) can be used to draw said regression between grain yield and stress index and to calculate the slope of said regression.

Drought effects on plants are usually mediated by more than one environmental factor. Soil water content, air moisture and heat are closely correlated. Dry periods are most of the time accompanied by dry air and high temperatures. It is thus advantageous to take more than one environmental parameter into account when determining drought tolerance. The inventors have found that, in that case, the relation between the grain yield and the various parameters is a multiple linear regression. Said multiple linear regression is given by a linear combination of the stress index for each of the parameters considered, wherein each of the index is affected by a coefficient. Said coefficients are represented by the individual drought tolerance values, i.e., the drought tolerance value which is obtained by considering only the regression between the grain yield and the stress index for the corresponding parameter. Such a linear combination of the index gives a regression whose quality is enhanced and/or which is more stable. In other words, the higher the number of environmental parameters introduced, the more accurate and representative across various locations and environmental conditions the regression obtained.

In a preferred embodiment, the regression is given by the equation:

$$\text{Grain Yield} = \mu + a_1 * W\_EP_1 + a_2 * W\_EP_2 + ... + a_m * W\_EP_m$$

Wherein $W\_EP_1$, $W\_EP_2$... $W\_EP_m$ each represents the stress index of one of m environmental parameters, and $a_1$, $a_2$, ..., $a_m$ represent the m corresponding m individual drought tolerance, m being an integer higher or equal to 2.

The slope of the resulting regression can then be determined, said slope being the drought tolerance of said plant line.

A "plant" according to the invention refers to any plant, particularly to agronomically useful plants (*e.g.,* seed plants). Preferably, the term "plant" includes whole plants, shoot vegetative organs/structures (*e.g.* leaves, stems and tubers), roots, flowers and floral organs/structures (*e.g.* bracts, sepals, petals, stamens, carpels, anthers and ovules), seeds (including embryo, endosperm, and seed coat), ear and fruits (the mature ovary), plant tissues (*e.g.* vascular tissue, ground tissue, and the like) and cells (*e.g.* guard cells, egg cells, trichomes and the like), and progeny of same.

The class of plants that can be used in the method of the invention is generally as broad as the class of higher and lower plants amenable to transformation techniques, including angiosperms (monocotyledonous and dicotyledonous plants), gymnosperms, ferns, and multicellular algae. It includes plants of a variety of ploidy levels, including aneuploid, polyploid, diploid, haploid and hemizygous. Included within the scope of the invention are all genera and species of higher and lower plants of the plant kingdom, for example wheat, sunflower, rape .... Included are furthermore the mature plants, seed, shoots and seedlings, and parts, propagation material (for example seeds and fruit) and cultures, for example cell cultures, derived therefrom.

Annual, perennial, monocotyledonous and dicotyledonous plants are preferred host organisms for the method according to the invention. Most preferably, the plant which can be used in the method of the invention is dicot or a monocot.

Preferably, the plant which is used in the method of the invention is maize (*Zea mays*).

As used herein, a "plant line" is a group of plants that display little or no genetic variation between individuals for at least one trait. Such lines may be created by taking advantage of the plant's method of pollination, e.g., self-pollination or cross-pollination. A plant is self-pollinated if pollen from one flower is transferred to the same or another flower of the same plant. A plant is cross-pollinated if the pollen comes from a flower on a different plant. Plants that have been self-pollinated and selected for type for many generations become homozygous at almost all gene loci and produce a uniform population of true breeding progeny. As used herein, the phrase "inbred line" refers to a genetically homozygous or nearly homozygous population. An inbred line, for example, can be derived through several cycles of brother/sister breedings or of selfing. In some embodiments, inbred lines breed true for one or more phenotypic traits of interest. An "inbred", "inbred individual", or "inbred progeny" is an individual sampled from an inbred line. A cross between two different homozygous lines produces a uniform population of hybrid plants that may be heterozygous for many gene loci. A cross of two plants each heterozygous at a number of gene loci will produce a "population of hybrid plants" that differ genetically and will not be uniform. As used herein, the terms "hybrid", "hybrid plant", and "hybrid progeny" refers to an individual produced from genetically different parents (e.g., a genetically heterozygous or mostly heterozygous individual).

In the context of the invention "plant line" refers to seeds from self-pollinated or cross pollinated plants at any step of the selection of the line or to a segregating population. But line could also refers to "inbred line" or "hybrid line". Hybrid line are future commercial hybrids or tested hybrid when an inbred line is test for is hybrid value by a cross with a tester line.

The drought tolerance of a "plant line" can be determined with the method of the invention. When the same method is applied to more than one plant line, the drought tolerance of each plant line can be determined. Said plants lines can be compared based on their respective drought tolerance values.

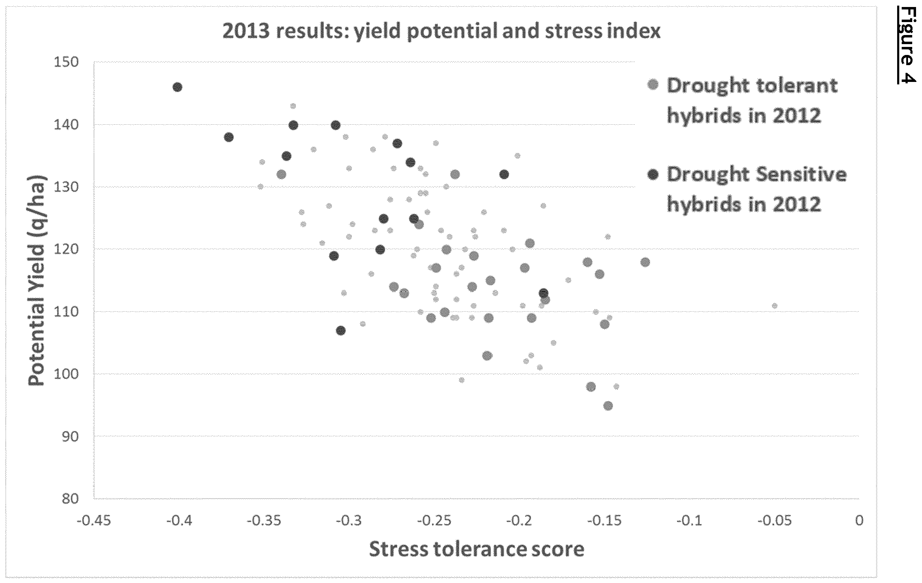

The method of the invention is particularly advantageous since it displays a good repeatability from one season to another. The inventors have shown that plant lines evaluated as tolerant (or sensitive) one year were still considered tolerant (or sensitive) the following year (see e.g. figure 4). Thus the results issued from the method lead to the same drought diagnostic for the two seasons. This validates the potential use of the method for predicting the drought tolerance of a maize line for which no information on grain yield and stress index is available because it has never been accurately measured, and herein defined as non-phenotyped maize line.

Thus in another aspect, the present invention is related to a method for predicting the drought tolerance of a non-phenotyped maize line based on the relatedness between said non-phenotyped maize line and its relative(s) for which the computing as defined in step d) of claim 1 was previously carried out.

In another aspect, the present invention is thus directed to a method for predicting the drought tolerance of a non-phenotyped maize line, said method comprising the computing as defined in step d) of claim 1 using a random model integrating information from the relative(s) of said maize line, and preferably including phenotypic records as well as molecular markers and/or pedigree.

The methods according to the present invention, for determining the drought tolerance or for predicting the drought tolerance of a non-phenotyped maize line, may be used for ranking plant lines and defining the commercial value of a plant line in regards to its drought tolerance.

Thus in another aspect, the present invention provides a method for comparing two or more plant lines, said method comprising a step of determining or predicting the drought tolerance or the drought stress tolerance of said plant lines as defined above. Preferably, a first plant line is more sensitive to drought than a second plant line if the drought tolerance value of said first plant line is inferior in absolute value to the drought tolerance value of said second plant line. Conversely, a first plant line is more resistant to drought stress than a second plant line if the drought tolerance value of said first plant line is superior in absolute value to the drought tolerance value of said second plant line.

This comparison enables ranking said plant lines based on their drought stress tolerance values. Thus, the invention also relates to a method of ranking two or more plant lines, said method comprising (a) determining the drought tolerance score of said plant lines and (b) ranking said plant lines based on their respective drought tolerance determined in (a). In particular, ranking plant lines can be interesting for selecting hybrid lines with enhanced agricultural and commercial value. In addition, such ranking can be important for selecting parent lines in a breeding program, as well as in evaluating the progeny obtained by said program.

Abiotic stress and among them, moisture deficits are one of the greatest challenges to future crop production. Although changes in tillage and irrigation practices can improve production by conserving water, enhancing the genetic tolerance of crops to drought stress through breeding is considered an essential strategy for addressing water deficits in particular because water consumption by maize culture if costly for the farmers and have a negative impact on public opinion on the agriculture. It is thus important to be capable of identifying plant lines with enhanced drought tolerance resulting from breeding programs. However, most phenotypic traits of interests, such as drought tolerance, are quantitative traits. As used herein, the phrase "quantitative trait" refers to a phenotypic trait that can be described numerically (i.e., quantitated or quantified). A quantitative trait typically exhibits continuous variation between individuals of a population; that is, differences in the numerical value of the phenotypic trait are slight and grade into each other. Frequently, the frequency distribution in a population of a quantitative phenotypic trait exhibits a bell-shaped curve (i.e., exhibits a normal distribution between two extremes). A quantitative trait is typically the result of a genetic locus interacting with the environment or of multiple genetic loci (QTL) interacting with each other and/or with the environment.

The drought tolerance as determined or predicted by the methods of the invention can be used as a trait to carry out genetic association study and to identify drought tolerance QTL.

Thus in another aspect, the invention relates to a method of identifying QTLs associated with drought tolerance in maize, said method comprising the steps of:

i) determining or predicting the drought stress tolerance of individuals from a maize population according to the methods of the invention; and

ii) identifying at least one genomic marker which is genetically linked with the drought stress tolerance determined in i).

Optionally, the method may comprise a prior step of crossing two or more parent plants. In this case, the population of steps i) and ii) is the progeny of said cross.

As an illustration, the inventors have found that 4 QTLs are associated with drought resistance in maize. As used herein, the term "quantitative trait locus" (QTL) refers to an association between a genetic marker and a chromosomal region and/or gene that affects the phenotype of a trait of interest. Typically, this is determined statistically; e.g., based on one or more methods published in the literature. A QTL can be a chromosomal region and/or a genetic locus with at least two alleles that differentially affect the expression of a phenotypic trait (either a quantitative trait or a qualitative trait). The presence of any of these QTLs is associated with enhanced drought tolerance, as determined by the method described above.

"Genetic linkage" is understood within the scope of the invention to refer to an association of characters in inheritance due to location of genes in proximity on the same chromosome, measured by percent recombination between loci (centi-Morgan, cM).

Each of the said marker loci can be easily identified through detection of the sequences bordering said marker locus. Said detection can be performed by any means known to the person of skills in the art. Methods for detecting a nucleic acid in a biological sample include *inter alia* hybridization with a labelled probe, amplification, including PCR amplification, sequencing, and all other methods known to the person of skills in the art. Detection of said sequences may be notably performed using well known technologies such as quantitative PCR or nucleic acid microarray technologies (including cDNA and oligonucleotide microarrays). These technologies are now used routinely by those skilled in the art and thus do not need to be detailed here.

The examples that follow are merely exemplary of the scope of this invention and content of this disclosure. One skilled in the art can devise and construct numerous modifications to the examples listed below without departing from the scope of this invention.

## FIGURE LEGENDS

Figure 1: Stress Index Method to characterize Maize Hybrids for drought tolerance. Hybrids performance is correlated with the intensity of the drought stress over the yield trails network. The slope of the regression is the drought tolerance score. For the first hybrid the slope is close to zero, the yield is sparsely affected by the stress, the hybrid is tolerant to stress, for the second hybrid yield is affected by drought stress, this hydrid is sensitive to stress.

Figure 2: Evaluation of hybrids for drought tolerance score and yield potential.

Figure 3: correlation between observed and estimated flowering dates in 2013. 85% of predicted values are within a 2-day difference from the observed values.

Figure 4: Classification of hybrids for stress tolerance score and yield potential: Hybrids evaluated as drought sensitive in 2012 are still sensitive in 2013 while hybrids evaluated as drought tolerant in 2012 are still tolerant in 2013.

## EXAMPLES

### 1. Background

The objective of a first study (Chapuis et al, 2012) was to assess corn hybrids for drought tolerance using the correlation between the level of stress and the seed number per plant. The level of stress was computed using soil tensiometers values averaged over different periods such as flowering and grain filling. The stress undergone by the plants was characterized by using only soil water probes values. Furthermore, only the stress index calculated during the flowering period was taken into account. Finally, the correlation between the level of stress and the yield was found as non-significant.

Seed number per plant is only a component of the final yield. The number of plants per acreage and the thousand kernel weight (TKW) are two other components to account for in the yields calculation. Indeed, the yield is equal to the seed number per acreage multiply by the TKW. There is no genotypic component in the number of plants per acreage.

### 2. Research approach

The present method aims at describing as well as possible the tolerance of maize lines (hybrid or inbred line) to abiotic stresses and more specifically todrought, including soil water deficit, air water deficit, and heat stress.

This method relies on two main aspects:

- Seizing the opportunity of the large trials network to sample a wide range of stress.

- The direct correlation between the yield (instead of intermediate yields components) and the level of stress.

In other words, one takes advantage of every single experiment location to evaluate one given line for stress tolerance. This leads to costs savings with limited specific drought experiments.

Plant breeding experiments, environmental monitoring

Stress tolerance evaluation relies on a yield trials network. A yield trial network corresponds to multiple geographically separated locations, on which a set of different genotypes is grown. Every experiment location is equipped with weather sensors, such as water probes, thermometers, water gauges and/or weather stations. These different sensors give information used to characterize the abiotic stresses undergone by the plants over the growing season. For example, the water probes monitor the soil water available for the crop. Drought stress, heat stress, air water stress, and cold stress are examples of abiotic stresses that may be taken into account. One example of yield network trial is given in Chapuis et al, 2012.

A database is used to store the information collected by the sensors from every experiment location. The data may then be organized for automated transfer to a centralized location for analysis such as central database. Data collected in this database is used by an analysis engine.

Trait phenotype measurements are collected, including morphological, phenological, physiological, crop growth traits. This also may include flowering times, yield and moisture at harvest. As for the sensors information, the trait phenotype measurements are stored in a database. The data may then be organized for automated transfer to a centralized location for analysis such as central database. Data collected in this database is used by an analysis engine.

The analysis engine extracts pertinent information from both environmental and trait phenotype databases and uses computation algorithms to compute the stress index based on environmental and phenological data.

The measurements collected by the sensors give information about the type of stress, its intensity and its period of occurrence. Related to the phenological stages of the plants, a stress index is computed for every line (inbred or hybrid) * location combination. This index is an accurate indicator of the intensity of the stress faced by the variety during the season. For example, one drought stress would have more consequences if it occurs at or after flowering than during the earlier vegetative stages. The algorithms to compute the stress index will be described later.

Definition of the most sensitive plant stages

This stress index is an indicator of the intensity of the drought stress at the most sensitive plant stages. As described hereafter, it is computed as a weighted combination of the averages of the soil water tensiometers values, the Air water stress and the heat stress indicator on two thermal time windows around the flowering and at grain filling. Thermal time is computed on degree days (dd), using the 10°C and 30°C thresholds. These two thermal time windows are described in Table 1.

Table 1: Thermal time windows for the two critical phenological periods

| Periods | Beginning | End |
|---|---|---|
| **Flowering** | Female flowering - 80 dd | Female flowering + 110 dd |
| **Grain Filling** | Female flowering + 110 dd | Grain Abortion limits stage Female flowering + 550 dd |

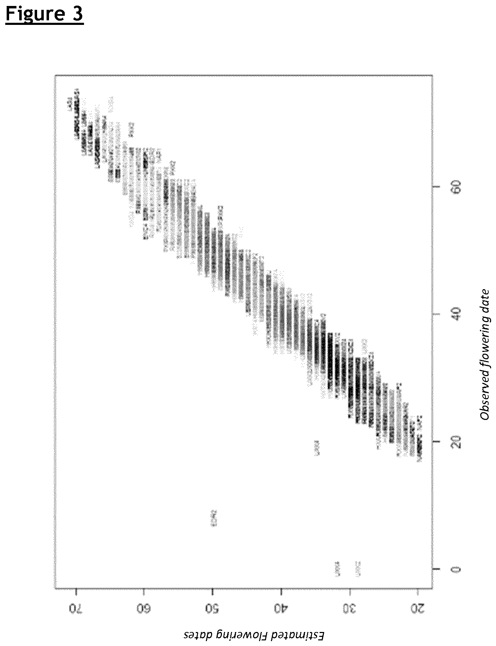

Thus, to describe with accuracy the intensity of the stress faced by a specific hybrid, the flowering date has to be set. This bypasses the bias due to confounding effect between escape and stress tolerance. Scoring flowering is time consuming and impossible at a large scale with tens of thousands of plots per location. To tackle this issue, a mixed approach has been developed, combining both phenotyping and modelling. This approach implies to score few genotypes and to model the remnant based on cumulated thermal time. Thus, on a subset of representative genotypes, female flowering measurements are taken on all locations of the yield trials network. For every genotype not included in this subset, female flowering is measured on at least one location. Currently, we are predicting the flowering dates of all genotypes by scoring only 10% of the trial plots. The Figure 3 illustrates the correlation between observed and estimated flowering dates in 2013. These predictions are then used to compute with accuracy the two thermal time windows as described above.

Data analysis

The drought stress includes different components, including as e.g., the soil water deficit, the air water deficit, the heat stress and light radiation. In order to accurately determining the drought tolerance, these components are first assessed individually. For each of these components, a stress index can thus be computed, which leads to the drought tolerance.

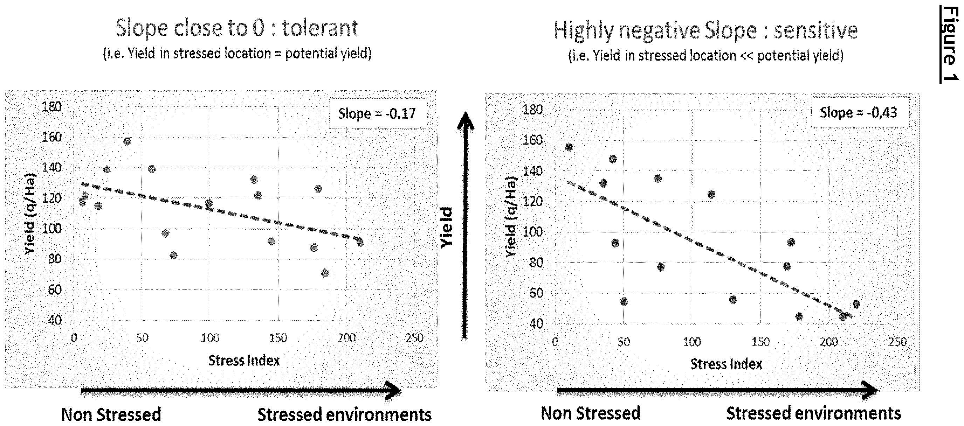

More specifically, for one hybrid tested on several locations, we obtain both a yield value and the stress index value for every location. Over the trials network, we draw the regression between the yield values and the stress index values for every hybrid. The slope of this linear regression describes the drought stress tolerance for one given hybrid. The more sensitive to drought stress a hybrid is, the lower its yield will be in stressed conditions, the higher the slope of the regression will be. At the opposite, a stress tolerant hybrid will have consistent yields across stressed conditions. Indeed, its regression slope will be close to zero (Figure 1). The trials network offers a wide range of conditions and optimizes the chance to get a large diversity of levels of stress. This wide range is required for the significance and the robustness of the regression. The quality of the regression is measured through the determination coefficient $R^2$ of the regression (yield over stress index). The better the stress index describes the environmental conditions, the higher the $R^2$ is.

The regression can be written as following for every genotype:

$$\text{Grain Yield} = \mu + a * \text{Stress Index (Eq. 1)}$$

with "$\mu$" being the yield for a stress index equal to 0, equivalent to the potential yield of the given hybrid in absence of stress; and "$a$" being the drought tolerance score for a given genotype

The yield, rather than its yield components such as the thousand kernel weight or the seed number, is preferred to compute the stress tolerance score. In fact, seed number would be a good indicator of the yield if the TKW was not variable between genotypes. On a panel of inbred lines testcrossed to a common tester, we perform an analysis of variance to test the genotype's effect on the TKW. This panel was phenotyped on three locations, with two levels of irrigation for each of this location (well-watered conditions and water limited conditions). The analysis of variance for thousand kernel weight as well as for grain yield indicated highly significant effects for sites and hybrids (Table 2). The TKW component, as the seed number per plant, is an important part to explain the grain yield. Plant breeding targets at improving the grain yield under abiotic stressed conditions. The drought evaluation of the varieties requires to be carried out on the final commercial trait of interest, which is the yield.

Table 2: Variance analysis of thousand kernel weight

| Source | Degree of freedom | Grain Yield well watered | Grain Yield Water limited | TKW well watered | TKW Water limited |
|---|---|---|---|---|---|
| **Location** | 2 | 5 958*** | 261 070*** | 393 782*** | 22 718*** |

(continued)

| Source | Degree of freedom | Grain Yield well watered | Grain Yield Water limited | TKW well watered | TKW Water limited |
|---|---|---|---|---|---|
| Hybrids | 282 | 209*** | 131*** | 842*** | 821*** |

*Mean Squares for thousand kernel weight (TKW) and Grain Yield in well watered and water limited conditions for the panel of hybrids evaluated on three locations, 2 water treatments per location; The "***" means a statistic significance at 0.001 for the corresponding source.*

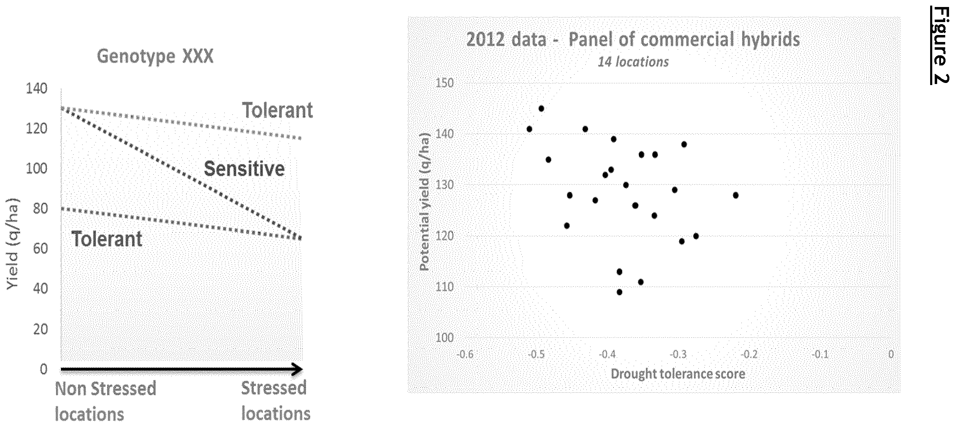

In combination to the potential yield, hybrids can be assessed based on their agronomic performances (potential yield) and their drought stress tolerance score (Figure 2).

### 3. Example 1: stress index based on soil water deficit only

Based on one thermal time window

In order to determine which phenological period is the most important for determining drought tolerance, a stress index was determined for each of said periods and the regression between this stress index and the yield drawn. These regressions may then be compared.

Flowering time is the most sensitive period to drought for maize. A stress index "IT_Flowering" may be computed using the average of the water probes values over the flowering time period.

The IT_Flowering index is computed using the following formula:

$$\text{IT\_Flowering} = \frac{1}{n}\sum_{t=Init}^{t=End} IT_t \quad \text{(Eq. 2)}$$

*with "Init" being the beginning and "End" the end of the Flowering period as defined in Table 1, "$IT_t$" being the value of the soil water probe at the time unit t, and "n" being the number of time units of the period. The time unit may be defined in day or in hour.*

In some situations depending on the locations or year effects, the drought stress occurs later and the most critical period is at grain filling. A stress index "IT_Fillering" may be computed using the average of the water probes values over the Grain filling time period.

In this scheme, the drought tolerance score "*a*" for a given genotype will be computed based on the following regression:

$$\text{Grain Yield} = \mu + a * \text{IT\_Period} \quad \text{(Eq. 3)}$$

The quality of the regression between these two stress index is compared for six sets of hybrids (Table 3). For each of these trial sets, all the hybrids constituting the set are tested on the same locations.

Table 3 summarizes the quality of the regression. On average, the IT_Filling based stress index is the most appropriate index to describe the stress occurring on the yield trials network. This tendency is especially strong in 2013, while the drought stress occurred mostly after the flowering of the corn plants. In 2012, the drought stress occurs earlier and the IT_Flowering stress index describes a good part of the grain yield's variation.

Table 3: Quality of regression for yield over stress index using IT_Flowering and IT_Filling

| Trial Set | Year of testing | Number of hybrids per set | Number of testing locations | $R^2$ Mean for IT_Flowering | $R^2$ Mean for IT_Filling |
|---|---|---|---|---|---|
| A | 2014 | 90 | 24 | 0.32 | 0.43 |
| B | 2014 | 38 | 26 | 0.36 | 0.28 |
| C | 2013 | 28 | 29 | 0.18 | 0.39 |
| D | 2013 | 23 | 26 | 0.14 | 0.38 |
| E | 2012 | 15 | 20 | 0.39 | 0.59 |

(continued)

| Trial Set | Year of testing | Number of hybrids per set | Number of testing locations | $R^2$ Mean for IT_Flowering | $R^2$ Mean for IT_Filling |
|---|---|---|---|---|---|
| F | 2012 | 13 | 23 | 0.41 | 0.68 |
| $R^2$ Mean is the average of every $R^2$ computed from the regression of the grain yield over the stress index for each hybrid of the trial set. | | | | | |

Based on a fixed weighted combination of the thermal time window

A posteriori, it is difficult to know when the drought stress will occur. To tackle this issue, it is decided to account for both the flowering and the grain filling periods. Thus the stress index is computed as a weighted combination of the averages of the soil water probes values on the two thermal time windows around the flowering and at grain filling. The stress index is thus described as:

$$\text{Stress Index} = \alpha * \text{IT\_Flowering} + \beta * \text{IT\_Filling} \quad (\text{Eq.4})$$

with $\alpha$ and $\beta$ being the weights, meeting the following condition $\alpha + \beta = 1$

In this scheme, the drought tolerance score "$a$" for a given genotype will be computed based on the following regression:

$$\text{Grain Yield} = \mu + a * (\alpha * \text{IT\_Flowering} + \beta * \text{IT\_Filling}) \quad (\text{Eq. 5})$$

According to the recommended scheme, $\alpha$ is equal to 2/3 and $\beta$ is equal to 1/3. These values give twice more importance to the flowering period than to the grain filling period. This is confirmed by the literature, stating the most critical phenological stage for sensitivity to drought during the flowering. This combination is used to compute the W_IT_1 stress index.

Nevertheless, other combinations of $\alpha$ and $\beta$ were tested, driven to a good fit of the yield over the stress index. A novel stress index, W_IT _2 uses $\alpha$ equal to 1/3 and $\beta$ equal to 2/3.

In an embodiment, other stress index can be derived by trying to maximize the variance of the slope of the response to the tested stress indices in a random regression model.

Alternatively, other stress indices can be derived by identifying proposed stress indices with a high correlation to the loadings of a factor analytic model used to characterize genotype by environments interaction patterns.

Table 4: Quality of regression for yield over stress index using W_IT_1 and W_IT_2

| Trial Set | Year of testing | Number of hybrids per set | Number of testing locations | $R^2$ Mean for IT_Flowering | $R^2$ Mean for IT_Filling | $R^2$ Mean for W_IT_1 | $R^2$ Mean for W_IT_2 |
|---|---|---|---|---|---|---|---|
| A | 2014 | 90 | 24 | 0.32 | 0.43 | 0.49 | 0.47 |
| B | 2014 | 38 | 26 | 0.36 | 0.28 | 0.33 | 0.31 |
| C | 2013 | 28 | 29 | 0.18 | 0.39 | 0.28 | 0.37 |
| D | 2013 | 23 | 26 | 0.14 | 0.38 | 0.25 | 0.35 |
| E | 2012 | 15 | 20 | 0.39 | 0.59 | 0.50 | 0.66 |
| F | 2012 | 13 | 23 | 0.41 | 0.68 | 0.56 | 0.70 |
| $R^2$ Mean is the average of every $R^2$ computed from the regression of the grain yield over the stress index for each hybrid of the trial set. | | | | | | | |

In most scenarii, the weighted combination of IT_Flowering and IT_Filling drives to a better quality of regression. In all situations, the weighted combination is better than the IT_Flowering alone. In few situations, the IT_Filling presents slightly better results than the weighted combination. But accounting for the two periods in the stress index enables to bypass the uncertainty related to the timing of the drought stress occurrence. Thus a common stress index is more stable over all locations and environmental conditions.

## 4. Example 2: stress Index based on Air water stress only

To gain in accuracy in the description of the stress index, an Air water stress index is computed over the same two thermal time windows around the flowering and at grain filling. The air water stress index is the average of the Vapor Pressure Deficit values over the two thermal time windows around the flowering and at grain filling.

The VPD based index is computed using the following formula:

$$VPD_{Period} = \frac{1}{n} \sum_{t=Init}^{t=End} VPD_t \qquad \text{(Eq. 6)}$$

with "Init" being the beginning and "End" the end of the period of interest as defined in Table 1, "$VPD_t$" being the value of the VPD at the time unit t, and "n" being the number of time units of the period. The time unit may be defined in day or in hour.

For comparison, Table 5 presents the quality of the regression using a VPD only based stress index computed at Flowering and at Grain Filling: VPD_Flowering and VPD_Filling.

Table 5: Quality of regression for yield over stress index using VPD_Flowering and VPD_Filling

| Trial Set | Year of testing | Number of hybrids per set | Number of testing locations | $R^2$ Mean for IT_ Flowering | $R^2$ Mean for IT_ Filling | $R^2$ Mean for VPD_ Flowering | $R^2$ Mean for VPD_Filling |
|---|---|---|---|---|---|---|---|
| A | 2014 | 90 | 24 | 0.32 | 0.43 | 0.29 | 0.60 |
| B | 2014 | 38 | 26 | 0.36 | 0.28 | 0.13 | 0.44 |
| C | 2013 | 28 | 29 | 0.18 | 0.39 | 0.13 | 0.42 |
| D | 2013 | 23 | 26 | 0.14 | 0.38 | 0.04 | 0.40 |
| E | 2012 | 15 | 20 | 0.39 | 0.59 | 0.65 | 0.55 |
| F | 2012 | 13 | 23 | 0.41 | 0.68 | 0.25 | 0.55 |
| $R^2$ Mean is the average of every $R^2$ computed from the regression of the grain yield over the stress index for each hybrid of the trial set. | | | | | | | |

As for the soil water deficit, index, a weighted combination of VPD_Flowering and VPD_Filling is used:

$$\text{Stress Index} = \gamma * \text{VPD\_Flowering} + \delta * \text{VPD\_Filling} \quad \text{(Eq. 7)}$$

with $\gamma$ and $\delta$ being the weights, meeting the following condition $\gamma + \delta = 1$.

In this scheme, the drought tolerance score "a" for a given genoytype will be computed based on the following regression:

$$\text{Grain Yield} = \mu + a * (\gamma * \text{VPD\_Flowering} + \delta * \text{VPD\_Filling}) \quad \text{(Eq. 8)}$$

According to the recommendations scheme, $\gamma$ is equal to 2/3 and $\delta$ is equal to 1/3. These values give twice more importance to the flowering period than to the grain filling period. This is confirmed by the literature, stating the most critical phenological stage for sensitivity to drought during the flowering.

This combination is used to compute the W_VPD_1 stress index.

Nevertheless, other combinations of $\gamma$ and $\delta$ were tested, driven to a good fit of the yield over the stress index. A novel stress index, W_VPD_2 uses $\gamma$ equal to 1/3 and $\delta$ equal to 2/3.

This reflects that for a given year, the main stress occurs at grain filling rather than at Flowering.

Table 6 presents the comparison of the quality of regression for the weighted combinations of the stress index computed for the soil water stress and for the air water stress.

Table 6: Quality of regression for yield over stress index using W_VPD_1 and W_VPD_2

| Trial Set | Year of testing | Number of hybrids per set | Number of testing locations | $R^2$ Mean for W_IT_1 | $R^2$ Mean for W_IT_2 | $R^2$ Mean for W_VPD_1 | $R^2$ Mean for W_VPD_2 |
|---|---|---|---|---|---|---|---|
| A | 2014 | 90 | 24 | 0.49 | 0.47 | 0.51 | 0.59 |
| B | 2014 | 38 | 26 | 0.33 | 0.31 | 0.29 | 0.39 |
| C | 2013 | 28 | 29 | 0.28 | 0.37 | 0.29 | 0.38 |
| D | 2013 | 23 | 26 | 0.25 | 0.35 | 0.19 | 0.33 |
| E | 2012 | 15 | 20 | 0.50 | 0.66 | 0.70 | 0.70 |
| F | 2012 | 13 | 23 | 0.56 | 0.70 | 0.32 | 0.45 |
| *$R^2$ Mean is the average of every $R^2$ computed from the regression of the grain yield over the stress index for each hybrid of the trial set.* | | | | | | | |

The VPD based stress index is a good indicator to describe the stress undergone by the plants. The quality of the regression is similar to the quality of the regression obtained with a soil water stress based index.

This result is not unexpected because soil water stress and Vapor Pressure Deficit are closely correlated. Dry periods are most of the time accompanied by high temperatures and dry air.

Nevertheless, these two indicators are partially complementary in the stress description.

In fact, the correlation between the stress tolerance score computed from the W_IT_2 and the stress tolerance score computed from the W_VDP_2 is more and less strong, depending of the year (related to specific environmental conditions). A lower correlation is not necessarily due to a lack of correlation between the two stress index W_IT_2 and W_VDP_2.

Table 7 summarizes these correlations.

Table 7: Correlations between the stress tolerance scores computed from W_IT_2 and W_VPD_2

| Trial Set | Year of testing | Number of hybrids per set | Number of testing locations | Correlation between W_IT_2 and W_VPD_2 stress index | Correlation between the drought tolerance scores computed from W_IT_2 and W_VPD_2 |
|---|---|---|---|---|---|
| A | 2014 | 90 | 24 | 0.83 | 0.56 |
| B | 2014 | 38 | 26 | 0.75 | 0.85 |
| C | 2013 | 28 | 29 | 0.56 | 0.48 |
| D | 2013 | 23 | 26 | 0.45 | 0.49 |
| E | 2012 | 15 | 20 | 0.89 | 0.35 |
| F | 2012 | 13 | 23 | 0.78 | 0.24 |

## 5. Example 3: stress Index based on soil water deficit and Air water stress

Based on previous results, combining Soil water deficit and Air water stress may be of interest to compute the stress index. In this situation, the integration of these two stresses may be done either by using the interaction "Tensiometric index * VPD" or by a linear combination of the previous W_IT and W_VPD indexes.

- The interaction index SoilAirStress is computed using the following formula:

$$Soil * Air_{Period} = \frac{1}{n} \sum_{t=Init}^{t=End} VPD_t * IT_t \text{ (Eq. 9)}$$

with "Init" being the beginning and "End" the end of the period of interest as defined in the Table 1, "$VPD_t$" being the value of the VPD at the time unit t, "$IT_t$" being the value of the water probes at the time unit t and "n" being the

*number of time units of the period.*

As previously, a linear combination of the index is computed using a weighted combinations of Soil*Air$_{Flowering}$ and Soil*Air$_{Filling}$. The values of the weights are defined following the same rules than previously.

In this scheme, the drought tolerance score "*a*" for a given genotype will be computed based on the following regression:

$$\text{Grain Yield} = \mu + a * (\text{W\_SoilAir}) \qquad (\text{Eq. 10})$$

This score will describe the overall stress tolerance of the genotype (without distinction between soil drought stress and air water drought stress tolerance).

- The linear combination of water soil deficit and Air water stress is computed using the following formula:

$$W\_Soil + Air = \sigma * \text{W\_IT} + \tau * W\_VPD \qquad (\text{Eq. 11})$$

*with $\sigma$ and $\tau$ being genotypes dependent. W_IT and W_VPD may be indifferently W_IT_1 or W_IT_2 and W_VPD_1 or W_VPD_2.*

In this scheme, $\sigma$ and $\tau$ are the individual drought tolerance scores for soil water deficit and air water deficit, respectively, for a given genotype. the regression grain yield and stress index is given by the following formula:

$$\text{Grain Yield} = \mu + \sigma * \text{W\_IT} + \tau * W\_VPD \qquad (\text{Eq. 12})$$

wherein the overall drought tolerance is given by the slope of the regression.

Table 8 summarizes the quality of the regression using both water deficit and air water stress. Whereas the integration of the two stresses is not always better in all situations, this brings stability in the quality of the regression. In other words, the soil water deficit only or the air water stress only may present a better quality of regression than the integration of both stress (trials set A and F). Nevertheless, this advantage depends on the specific environmental conditions and a stable stress index across different conditions is highly preferred. This is to meet the objective to compute a stress index that is the most stable over different environmental situations.

In terms of quality of the regression, there is no significant difference between the linear combination and the interaction of the soil water stress and the air water stress.

Table 8: Quality of regression for yield over stress index using water deficit, air water stress and heat stress

| Trial Set | Year of test | $R^2$ Mean for W_IT_2 | $R^2$ Mean for W_VPD_2 | $R^2$ Mean for W_Soil*Air_2 | $R^2$ Mean for W_Soil+Air_2 | $R^2$ Mean for W_SAH |
|---|---|---|---|---|---|---|
| A | 2014 | 0.47 | 0.59 | | 0.58 | 0.62 |
| B | 2014 | 0.31 | 0.39 | | 0.38 | 0.44 |
| C | 2013 | 0.37 | 0.38 | 0.44 | 0.49 | 0.60 |
| D | 2013 | 0.35 | 0.33 | 0.50 | 0.53 | 0.67 |
| E | 2012 | 0.66 | 0.70 | 0.75 | 0.70 | 0.74 |
| F | 2012 | 0.70 | 0.45 | 0.39 | 0.58 | 0.62 |
| *$R^2$ Mean is the average of every $R^2$ computed from the regression of the grain yield over the stress index for each hybrid of the trial set.* | | | | | | |

## 6. Example 4: stress Index based on soil water deficit, Air water stress and heat stress

The heat stress may be of interest to describe at the best the abiotic stresses occurring in every environment. The heat stress based index may be computed using the following formula:

$$Heat_{Period} = \frac{1}{n}\sum_{t=Init}^{t=End}( O \ if \ Tmax_t < T , Tmax_t\_T \ if \ Tmax_t > T) \quad \text{(Eq. 13)}$$

with "Init" being the beginning and "End" the end of the period of interest as defined in Table 1, "$Tmax_t$" being the daily maximum temperature at the time unit t, "T" being a temperature threshold and "n" being the number of time units of the period.

The temperature threshold is defined as the higher temperature, above which the heat stress occurs. We set up this threshold at 32°C. Nevertheless, this threshold may be at any value between 25°C and 35°C.

The heat stress may also be computed using the daily minimum temperature. In this case, the heat stress based index may be computed using the following formula. The threshold may be defined at any level between 18°C and 25°C.

$$Heat_{Period} = \frac{1}{n}\sum_{t=Init}^{t=End}( O \ if \ Tmin_t < T , Tmin_t\_T \ if \ Tmin_t > T) \quad \text{(Eq. 14)}$$

with "Init" being the beginning and "End" the end of the period of interest as defined in the Table 1, "$Tmin_t$" being the daily maximum temperature at the time unit t, "T" being a temperature threshold and "n" being the number of time units of the period.

As previously, a linear combination of the index is computed using a weighted combination of $Heat_{Flowering}$ and $Heat_{Filling}$ to obtain a W_Heat index, which can be either W_Heat_1 or W_Heat_2. The values of the weights are defined following the same rules than previously.

The integration of the heat stress is done using a linear combination of the previous W_IT, W_VPD and W_Heat indexes.

$$W\_SAH = \sigma * W\_IT + \tau * W\_VPD + \theta * W\_Heat \quad \text{(Eq. 15)}$$

with $\sigma$, $\tau$ and $\theta$ being genotypes dependent. W_IT, W_VPD and W_Heat may be indifferently W_IT_1 or W_IT_2, W_VPD_1 or W_VPD_2 and W_Heat_1 or W_Heat_2.

In this scheme, $\sigma$, $\tau$ and $\theta$ are consecutively the soil drought tolerance, the Air water stress drought tolerance and the heat stress drought tolerance scores for a given genotype and are computed based on the following regression:

$$\text{Grain Yield} = \mu + \sigma * W\_IT + \tau * W\_VPD + \theta * W\_Heat \quad \text{(Eq. 16)}$$

Table 8 summarizes the quality of the regression using these three stress. By adding the heat stress in the computation of the overall stress index, the quality of the regression is enhanced.

Summary of the computation of the stress index

Table 9 gathers the quality of the regression per year. This quality is described by the average $R^2$ of the regression of every genotype tested the given year. To be included into the summary, it was requested that a given genotype was tested at least on eight geographically separated locations.

The stress index is improved in comparison to a soil water deficit only based index computed at Flowering when:

- Both Flowering and grain Filling periods are taken into account;

- The Air water stress is introduced; and

- The heat stress is added to the soil water deficit and the air water stress.

Table 9: Summary table regarding the quality of regression for yield over major stress index using water deficit, air water stress and heat stress

| Year of test | Number of genoty pes | $R^2$ Mean IT_ Flowering | $R^2$ Mean for W_ IT_2 | $R^2$ Mean for W_ VPD_2 | $R^2$ Mean for W_Soil*Air_2 | $R^2$ Mean for W_Soil+Air_2 | $R^2$ Mean for W_SAH_2 |
|---|---|---|---|---|---|---|---|
| 2014 | 867 | 0.21 | 0.28 | 0.35 | | 0.38 | 0.53 |
| 2013 | 437 | 0.10 | 0.17 | 0.17 | 0.50 | 0.53 | 0.67 |
| 2012 | 191 | 0.36 | 0.50 | 0.41 | 0.34 | 0.45 | 0.56 |
| $R^2$ Mean is the average of every $R^2$ computed from the regression of the grain yield over the stress index for each hybrid. | | | | | | | |

### 7. Example 5: QTL analysis on a biparental population

The drought stress tolerance scores obtained using any of the Equations 3, 5, 8, 10, 12 and 16 can be used to run genetic association analysis. The objective of this analysis is to link favorable alleles carried by some individuals with appropriate stress tolerance behavior.

A biparental population of 258 individuals was testcrossed to a tester. The produced hybrids were tested on nine geographically separated locations in 2014.

Every individual was genotyped on an Affimetrix chip, with 7729 polymorphism SNP markers, evenly distributed across the genome.

A QTL analysis using the Composite Interval Mapping was carried out. The traits of interest were the grain yield, the grain moisture at harvest, the adjusted grain yield to moisture and the drought tolerance score as computed in Eq. 5. We use a threshold for the LOD score at 3 to identify QTL having a significant effect on the drought tolerance score.

Table 10 summarizes the results of this analysis. Four QTL were identified with a LOD score higher than 3. One of this QTL is collocated with a QTL for grain moisture, grain yield and adjusted grain yield in non-stressed conditions.

Table 10: QTL identified in this study for the drought tolerance score

| N° | CHROMOSOME | PIC. LOD | PIC.POS | LEFT.POS | RIGHT.POS | Other parameters |
|---|---|---|---|---|---|---|
| 1 | 1 | 3.56 | 440.88 | 434.59 | 444.43 | |
| 2 | 3 | 4.03 | 97.14 | 93.14 | 101.14 | |
| 3 | 3 | 6.77 | 206.73 | 199.34 | 206.73 | Grain Yield, Adjusted Grain Yield and Grain Moisture in non-stressed conditions |
| 4 | 8 | 4.03 | 88.37 | 82.37 | 93.40 | |

The value of the slope is closely dependent of the interactions genotype * environment. A hybrid that is capable to maintain its yield in stressed environments, has a slope close to 0. At the opposite, a hybrid that is high yielding in non-stressed environments with an average or low yield in stressed environments, has a larger negative value for the slope. Thus, it is expected to detect common QTL for the slope and for the yield in specific environments (stressed OR not stressed). Through, a constitutive QTL for the yield (i.e. detected whatever the intensity of the stress is), is not expected to be collocating with a QTL for the slope.

In other words, based on this theoretical approach, having common QTL for the slope and the yield in specific conditions validates the accuracy of our methodology for characterizing genotypes for stress tolerance.

In addition of the slope, 2 QTL analysis were carried out on the yield in stressed environments and on the yield in non-stressed environments. The data set was split into 2 subsets: one regrouping the highly stressed locations (W_IT2 > 100; 3 locations) and one regrouping the non-stressed locations (W_IT2 < 50; 3 locations). The 2 locations with a moderate stress level were taken away from the analysis. 4 QTL with a LOD value greater than 3 were detected. Among them, one QTL on chromosome 1 was detected in both stressed and non-stressed environments (LOD value = 5.6 in stressed and LOD value = 6 in non-stressed environments).

But, the QTL with the higher LOD (LOD value = 10.7) was detected on chromosome 3 only in non-stressed

# EP 3 304 377 B1

environments. This QTL collocates with the QTL3 for the slope and is the QTL that explains the more variance for both the slope and the yield. This is a validation that the stress index is a tool to characterize and rank the genotypes for stress tolerance.

In addition, the method enables to differentiate the stress tolerance depending of the type of stress (nature of the stress or phenological stage). Carrying out the QTL analysis on the drought tolerance score computed from an individual stress index gives information about specific tolerance. Indeed, some QTL can refer to specific tolerance to the VPD stress (Flowering or at Grain Filling) and others refer to a specific tolerance to a drought stress occurring at Grain Filling.

## 8. Example 6: Genetic association mapping on a panel of lines.

A panel of 284 inbred lines were testcrossed to a common tester. The produced hybrids were tested on 13 conditions. One condition is a combination of location * year * irrigation treatment. The irrigation treatment may be well watered condition or water-limited condition.

Every individual was genotyped on an Affimetrix 50K chip, with SNP markers, evenly distributed across the genome.

An association mapping study was carried out using mixed linear model. The traits of interest were the grain yield, the grain moisture at harvest, the adjusted grain yield to moisture and the drought tolerance score as computed in Eq. 3 and in Eq. 5. A threshold for the -log10 (pvalue) at 3 was used to identify genetic markers having a significant effect on the drought tolerance score.

The Table 11 summarizes the results of this analysis. Six zones were identified as significant for at least one of the drought tolerance score computed from Eq. 3 and Eq. 5. Three of these zones are collocated with zones identified for grain yield or adjusted grain yield.

Table 11: Markers with significant effect on the drought tolerance scores

| N° | CHR. | PIC.POS | -log10 (pvalue) | Traits | Other parameters |
|---|---|---|---|---|---|
| 1 | 1 | 59 | 3.4 | W_IT | Grain Yield |
| 2 | 2 | 340 | 3.4 | W_IT, IT_Filling | |
| 3 | 3 | 105 | 4.4 | IT_Filling | |
| 4 | 5 | 74 | 3 | IT_Filling | |
| 5 | 5 | 181 | 4.1 | IT_Filling, IT_Flowering | Grain Yield |
| 6 | 5 | 33 | 3.6 | W_IT, IT_Filling, IT_Flowering | Grain Yield |

LIST OF REFERENCES

Chapuis et al., 2012, Europ. J. Agronomy 42, 59-67
Bolanos and Edmeades, 1993, Field Crops Res. 31, 233-252
Bruce et al., 2002, J. Exp. Bot. 53, 13-25
Duvick, 2005, Adv. Agron. 86, 83-145
Messina et al., 2011, J. Exp. Bot. 62, 855-868
Tardieu and Tuberosa, 2010, Curr. Opin. Plant Biol. 13, 206-212
Tardieu et al., 2014, Plant Physiology 164(4), 1628-1635
Welcker et al., 2011, Plant Physiology 157(2), 718-729
Schaeffer, 2004, Livestock Production Science 86, 35-45
Lillehammer et al., 2007, Genet. Sel. Evol. 39, 105-121

**Claims**

1. A method for determining the drought tolerance of a maize line, said method comprising the steps of:

   a) Sowing said maize line in multiple locations;

b) Determining a stress index for each location of step a) wherein determining a stress index involves measuring one environmental parameter at regular intervals during a period of time around flowering time and during a period of time covering the grain filling;

c) Measuring grain yield of said maize line for each location of step a);

d) Computing a linear regression between the stress index of step b) and the grain yield of step c);

e) Determining drought tolerance based on the regression of step d).

2. The method according to claim 1, wherein said linear regression in step (d) is a linear regression with random effect(s) integrating information from the relative(s) of said maize line including phenotypic records as well as molecular markers and/or pedigree.

3. The method of any one of claims 1 to 2, wherein said stress index is the average of the measurements of said environmental parameter per time unit during said period of time.

4. The method of any one of claims 1 to 3, wherein said stress index is given by the equation:

$$\text{Stress Index} = W\_EP = \alpha * EP\_Flowering + \beta * EP\_Filling$$

wherein:

$$EP\_Flowering = \frac{1}{n} \sum_{t=Init}^{t=End} EP_t$$

with "Init" being the beginning and "End" the end of the flowering period, "EPt" the value of said environmental parameter measurement at the time unit t, and "n" the number of time units of the period; and

$$EP\_Filling = \frac{1}{n} \sum_{t=Init}^{t=End} EP_t$$

with "Init" being the beginning and "End" the end of the grain filling period, "EPt" the value of said environmental parameter measurement at the time unit t, and "n" the number of time units of the period; and

$\alpha$ and $\beta$ are the weights of the combination, with $\alpha + \beta = 1$.

5. The method of claim 4, wherein the value of $\alpha$ and $\beta$ is calculated by calculating the stress index for a year of experiment on a sample of known lines and determining the best $\alpha$ and $\beta$ value for this year of experiment.

6. The method of any one claims 4 or 5, wherein the value of $\alpha$ and $\beta$ is calculated by calculating the stress index for a sample of lines and using a crop model to help in determining the best $\alpha$ and $\beta$ values to apply on the full experiment.

7. The method of any one of claims 1 to 6, wherein the regression of step d) is given by the equation:

$$\text{Grain Yield} = \mu + a * W\_EP$$

wherein $\mu$ is the yield for a stress index equal to 0, equivalent to the potential yield of the given hybrid in absence of stress

and a is the drought tolerance.

8. The method of any one of claims 1 to 7, wherein determining said stress index involves measuring at least one other environmental parameter.

9. The method of any one of the claims 1 to 8, wherein he regression of step d) is given by the equation:

$$\text{Grain Yield} = \mu + a_1 * \text{W\_EP}_1 + a_2 * \text{W\_EP}_2 + ... + a_m * \text{W\_EP}_m$$

wherein $\text{W\_EP}_1$, $\text{W\_EP}_2$... $\text{W\_EP}_m$ each represents the stress index of one of m environmental parameters, and $a_1$, $a_2$, ..., $a_m$ represent the corresponding m individual drought tolerance of each environmental parameters, m being an integer equal to or higher than 2.

10. The method of any one of claims 1 to 9, wherein said environmental parameter is selected in the list consisting of the soil water index, the vapor pressure deficit, heat stress, and light radiation.

11. A method for predicting the drought tolerance of a non-phenotyped maize line, said method comprising the computing as defined in step d) of claim 1 using a random model integrating information from the relative(s) of said maize line, preferably including phenotypic records as well as molecular markers and/or pedigree.

12. A method for comparing two or more plants lines, said method comprising a step of determining or predicting the drought tolerance of said plants lines according to the method of any one of claims 1 to 11.

13. A method of identifying QTLs associated with drought tolerance in maize, said method comprising the steps of:

i) determining or predicting the drought tolerance of individuals from a maize population according to any one of claims 1-11;
ii) identifying at least one genomic marker which is genetically linked with the drought tolerance determined in i).

**Patentansprüche**

1. Verfahren zum Bestimmen der Trockenheitstoleranz einer Maissorte, wobei das Verfahren die folgenden Schritte umfasst:

a) Aussäen der Maissorte an mehreren Stellen;
b) Bestimmen eines Stressindex für jede Stelle von Schritt a), wobei das Bestimmen eines Stressindex das Messen eines Umweltparameters in regelmäßigen Abständen während eines Zeitabschnitts um die Blütezeit herum und während eines Zeitabschnitts einschließt, der die Kornfüllung umfasst;
c) Messen der Kornausbeute der Maissorte für jede Stelle von Schritt a);
d) Berechnen einer linearen Regression zwischen dem Stressindex von Schritt b) und der Kornausbeute von Schritt c);
e) Bestimmen der Trockenheitstoleranz auf der Basis der Regression von Schritt d).

2. Verfahren nach Anspruch 1, wobei die lineare Regression in Schritt (d) eine lineare Regression mit Zufallseffekt(en) ist, die Informationen von dem/den Verwandten der Maissorte integriert, einschließlich der phänotypischen Aufzeichnungen sowie molekularer Marker und/oder des Stammbaums.

3. Verfahren nach einem der Ansprüche 1 bis 2, wobei der Stressindex der Durchschnitt der Messungen des Umweltparameters pro Zeiteinheit während des Zeitabschnitts ist.

4. Verfahren nach einem der Ansprüche 1 bis 3, wobei der Stressindex durch die folgende Gleichung gegeben ist:

Stressindex = W_EP = $\alpha *$ EP_Blüte + $\beta *$ EP_Füllung wobei:

$$\text{EP\_Blüte} = \frac{1}{n} \sum_{t=Beginn}^{t=Ende} EP_t$$

wobei "Beginn" der Beginn und "Ende" das Ende der Blütezeit ist, "EPt" ist der Wert der Umweltparametermessung zur Zeiteinheit t, und "n" ist die Zahl der Zeiteinheiten der Periode; und

$$EP\_Füllung = \frac{1}{n} \sum_{t=Beginn}^{t=Ende} EP_t$$

wobei "Beginn" der Beginn und "Ende" das Ende der Kornfüllperiode ist, "EPt" ist der Wert der Umweltparametermessung zur Zeiteinheit t, und "n" ist die Zahl der Zeiteinheiten der Periode; und
$\alpha$ und $\beta$ sind die Gewichte der Kombination, mit $\alpha + \beta = 1$.

5. Verfahren nach Anspruch 4, wobei der Wert von $\alpha$ und $\beta$ berechnet wird durch Berechnen des Stressindex für ein Jahr des Experiments an einer Probe von bekannten Sorten und Bestimmen des besten $\alpha$- und $\beta$-Wertes für dieses Experimentjahr.

6. Verfahren nach einem der Ansprüche 4 oder 5, wobei der Wert von $\alpha$ und $\beta$ berechnet wird durch Berechnen des Stressindex für eine Probe von Sorten und Verwenden eines Erntemodells, um bei der Bestimmung der besten $\alpha$- und $\beta$-Werte zu helfen, um sie bei dem vollen Experiment anzuwenden.

7. Verfahren nach einem der Ansprüche 1 bis 6, wobei die Regression von Schritt d) gegeben ist durch die Gleichung:

$$Kornausbeute = \mu + a * W\_EP$$

wobei $\mu$ die Ausbeute für einen Stressindex gleich 0 ist, äquivalent zur potentiellen Ausbeute des gegebenen Hybrids bei Fehlen von Stress
und a ist die Trockenheitstoleranz.

8. Verfahren nach einem der Ansprüche 1 bis 7, wobei das Bestimmen des Stressindex das Messen von mindestens einem anderen Umweltparameter beinhaltet.

9. Verfahren nach einem der Ansprüche 1 bis 8, wobei die Regression von Schritt d) gegeben ist durch die Gleichung:

$$Kornausbeute = \mu + a_1 * W\_EP_1 + a_2 * W\_EP_2 + \ldots + a_m * W\_EP_m$$

wobei $W\_EP_1$, $W\_EP_2$ ... $W\_EP_m$ jeweils den Stressindex von einem von m Umweltparametern repräsentiert, und $a_1$, $a_2$, ..., $a_m$, repräsentiert die entsprechende m individuelle Trockenheitstoleranz von jedem Umweltparameter, wobei m eine Ganzzahl gleich oder größer als 2 ist.

10. Verfahren nach einem der Ansprüche 1 bis 9, wobei der Umweltparameter in der Liste ausgewählt wird, die besteht aus dem Bodenwasserindex, dem Dampfdruckdefizit, dem Wärmestress und der Lichtstrahlung.

11. Verfahren zum Vorhersagen der Trockenheitstoleranz einer nicht phänotypisierten Maissorte, wobei das Verfahren das Berechnen umfasst, wie in Schritt d) von Anspruch 1 festgelegt, unter Verwendung eines Zufallsmodells, das Informationen von dem/den Verwandten der Maissorte integriert, vorzugsweise einschließlich phänotypischer Aufzeichnungen sowie molekularer Marker und/oder des Stammbaums.

12. Verfahren zum Vergleichen von zwei oder mehr Pflanzensorten, wobei das Verfahren einen Schritt des Bestimmens oder der Vorhersage der Trockenheitstoleranz der Pflanzensorten entsprechend dem Verfahren nach einem der Ansprüche 1 bis 11 umfasst.

13. Verfahren zum Identifizieren von QTLs, die mit der Trockenheitstoleranz bei Mais verbunden sind, wobei das Verfahren die folgenden Schritte umfasst:

i) Bestimmen oder Vorhersagen der Trockenheitstoleranz von Individuen aus einer Maispopulation nach einem der Ansprüche 1 bis 11;
ii) Identifizieren von mindestens einem Genommarker, der genetisch mit der Trockenheitstoleranz verbunden ist, die in i) bestimmt wurde.

**Revendications**

1.  Procédé pour déterminer la tolérance à la sécheresse d'une lignée de maïs, ledit procédé comprenant les étapes suivantes :

    a) ensemencement de ladite lignée de maïs dans de multiples emplacements ;
    b) détermination de l'indice de stress pour chaque emplacement de l'étape a), dans laquelle la détermination de l'indice de stress implique la mesure d'un paramètre environnemental à des intervalles réguliers durant une période de temps aux alentours de la floraison et durant une période de temps couvrant le remplissage des grains ;
    c) mesure du rendement en grains de ladite lignée de maïs pour chaque emplacement de l'étape a) ;
    d) calcul d'une régression linéaire entre l'indice de stress de l'étape b) et le rendement en grains de l'étape c) ;
    e) détermination de la tolérance à la sécheresse sur la base de la régression de l'étape d).

2.  Procédé selon la revendication 1, dans lequel ladite régression linéaire dans l'étape (d) est une régression linéaire avec un ou plusieurs effets aléatoires intégrant des informations provenant de la parentèle de ladite lignée de maïs comprenant des enregistrements phénotypiques ainsi que des marqueurs moléculaires et/ou le pédigrée.

3.  Procédé selon l'une quelconque des revendications 1 à 2, dans lequel ledit indice de stress est la moyenne des mesures dudit paramètre environnemental par unité de temps durant ladite période de temps.

4.  Procédé selon l'une quelconque des revendications 1 à 3, dans lequel ledit indice de stress est donné par l'équation :

    Indice de stress = W_EP = $\alpha$ * EP_floraison + $\beta$ * EP_remplissage
    dans laquelle :

    $$EP\_floraison = \frac{1}{n} \sum_{t=début}^{t=fin} EP_t$$

    où « début » indique le début et « fin » indique la fin de la période de floraison, « EPt » indique la valeur de ladite mesure de paramètre environnemental à l'unité de temps t, et « n » indique le nombre d'unités de temps de la période ; et

    $$EP\_remplissage = \frac{1}{n} \sum_{t=début}^{t=fin} EP_t$$

    où « début » indique le début et « fin » indique la fin de la période de remplissage des grains, « EPt » indique la valeur de ladite mesure de paramètre environnemental à l'unité de temps t, et « n » indique le nombre d'unités de temps de la période ; et
    $\alpha$ et $\beta$ sont les poids de la combinaison, avec $\alpha + \beta = 1$.

5.  Procédé selon la revendication 4, dans lequel les valeurs $\alpha$ et $\beta$ sont calculées par calcul de l'indice de stress pour une année d'expérience sur un échantillon de lignées connues et détermination des meilleurs valeurs $\alpha$ et $\beta$ pour cette année d'expérience.

6.  Procédé selon l'une quelconque des revendications 4 ou 5, dans lequel les valeurs $\alpha$ et $\beta$ sont calculées par calcul de l'indice de stress pour un échantillon de lignées et utilisation d'un modèle de culture pour aider à déterminer les meilleures valeurs $\alpha$ et $\beta$ à appliquer sur toute l'expérience.

7.  Procédé selon l'une quelconque des revendications 1 à 6, dans lequel la régression de l'étape d) est donnée par l'équation :

    ```
    Rendement en grains = µ + a * W_EP
    ```

dans laquelle μ est le rendement pour un indice de stress égal à 0, équivalant au rendement potentiel de l'hybride donné en l'absence de stress
et a est la tolérance à la sécheresse.

8. Procédé selon l'une quelconque des revendications 1 à 7, dans lequel la détermination dudit indice de stress implique la mesure d'au moins un autre paramètre environnemental.

9. Procédé selon l'une quelconque des revendications 1 à 8, dans lequel la régression de l'étape d) est donnée par l'équation :

$$\text{Rendement en grains} = \mu + a_1 * W\_EP_1 + a_2 * W\_EP_2 + \ldots + a_m * W\_EP_m$$

dans laquelle chacun parmi $W\_EP_1$, $W\_EP_2$ ... $W\_EP_m$ représente l'indice de stress de l'un parmi m paramètres environnementaux, et $a_1$, $a_2$, ..., $a_m$ représentent la tolérance à la sécheresse individuelle correspondante de chacun des paramètres environnementaux, m étant un entier égal ou supérieur à 2.

10. Procédé selon l'une quelconque des revendications 1 à 9, dans lequel ledit paramètre environnemental est choisi dans la liste constituée par l'indice hydrique du sol, le déficit de pression de vapeur, le stress de chaleur, et le rayonnement lumineux.

11. Procédé pour prédire la tolérance à la sécheresse d'une lignée de maïs non phénotypée, ledit procédé comprenant le calcul tel que défini dans l'étape d) de la revendication 1 utilisant un modèle aléatoire intégrant des informations provenant de la parentèle de ladite lignée de maïs, incluant de préférence des enregistrements phénotypiques ainsi que des marqueurs moléculaires et/ou le pédigrée.

12. Procédé pour comparer deux ou plusieurs lignées de plantes, ledit procédé comprenant une étape de détermination ou de prédiction de la tolérance à la sécheresse desdites lignées de plantes conformément au procédé de l'une quelconque des revendications 1 à 11.

13. Procédé pour identifier des QTL associés à la tolérance à la sécheresse dans le maïs, ledit procédé comprenant les étapes suivantes :

i) détermination ou prédiction de la tolérance à la sécheresse d'individus provenant d'une population de maïs selon l'une quelconque des revendications 1 à 11 ;
ii) identification d'au moins un marqueur génomique qui est génétiquement lié à la tolérance à la sécheresse déterminée en i).

# Figure 1

Slope close to 0 : tolerant
(i.e. Yield in stressed location = potential yield)

Highly negative Slope : sensitive
(i.e. Yield in stressed location << potential yield)

# Figure 2

## Figure 3

## Figure 4

2013 results: yield potential and stress index

Drought tolerant hybrids in 2012

Drought Sensitive hybrids in 2012

Potential Yield (q/ha)

Stress tolerance score

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHAPUIS et al.** *Europ. J. Agronomy,* 2012, vol. 42, 59-67
- **BOLANOS ; EDMEADES.** *Field Crops Res.,* 1993, vol. 31, 233-252
- **BRUCE et al.** *J. Exp. Bot.,* 2002, vol. 53, 13-25
- **DUVICK.** *Adv. Agron.,* 2005, vol. 86, 83-145
- **MESSINA et al.** *J. Exp. Bot.,* 2011, vol. 62, 855-868
- **TARDIEU ; TUBEROSA.** *Curr. Opin. Plant Biol.,* 2010, vol. 13, 206-212
- **TARDIEU et al.** *Plant Physiology,* 2014, vol. 164 (4), 1628-1635
- **WELCKER et al.** *Plant Physiology,* 2011, vol. 157 (2), 718-729
- **SCHAEFFER.** *Livestock Production Science,* 2004, vol. 86, 35-45
- **LILLEHAMMER et al.** *Genet. Sel. Evol.,* 2007, vol. 39, 105-121